# EUROPÄISCHE PATENTSCHRIFT

(11) **EP 1 942 863 B1**
(45) Veröffentlichungstag und Bekanntmachung des Hinweises auf die Patenterteilung: **16.08.2017**
(21) Anmeldenummer: 06807425.1
(22) Anmeldetag: 20.10.2006
(51) Int. Cl.: A61Q 19/10, A61K 8/34, A61K 8/81, A61K 8/02

(54) **KOSMETISCHE GELFÖRMIGE WASCHAKTIVE ZUBEREITUNG ENTHALTEND 1,2-ALKANDIOL(EN) UND EIN ODER MEHRERE POLYACRYLATVERDICKER.**
COSMETIC, GEL-TYPE WASH-ACTIVE PREPARATION CONTAINING 1,2-ALKANEDIOL(S) AND ONE OR MORE POLYACRYLATE THICKENERS
PREPARATION COSMETIQUE, SOUS FORME DE GEL, DETERGENTE, CONTENANT DU 1,2-ALCANDIOL(ENE) ET UN OU PLUSIEURS EPAISSISSANTS DE POLYACRYLATE

(30) Priorität: 25.10.2005 DE 102005051866
(43) Veröffentlichungstag der Anmeldung: 16.07.2008
(73) Patentinhaber: Beiersdorf AG, 20253 Hamburg (DE)
(72) Erfinder: NIELSEN, Jens, 24558 Henstedt-Ulzburg (DE); BLECKMANN, Andreas, 22926 Ahrensburg (DE); ARGEMBEAUX, Horst, 21465 Wentorf (DE); DETERT, Marion, 22455 Hamburg (DE); RUPPERT, Stephan, 20259 Hamburg (DE); ALBRECHT, Harald, 8320 Fehraltorf (CH); KÜTHER, Jörg, 25469 Halstenbek (DE); AECHTNER, Anja, 68165 Mannheim (DE); THOMPSON, Susan, Chelmsford CM1 2NF (GB); KÖHLER, Manuela, 22147 Hamburg (DE)
(86) Internationale Anmeldenummer: PCT/EP2006/067604
(87) Internationale Veröffentlichungsnummer: WO 2007/048755

(56) Entgegenhaltungen:
- EP-A1- 1 426 029
- WO-A1-01/19946
- DE-T2- 69 713 904
- DATABASE EPODOC EUROPEAN PATENT OFFICE, THE HAGUE, NL; 28. April 2005 (2005-04-28), KAO CORP: "Detergent Composition" XP002413295 & JP 2005 113067 A (KAO CORP) 28. April 2005 (2005-04-28)
- DATABASE EPODOC EUROPEAN PATENT OFFICE, THE HAGUE, NL; 28. April 2005 (2005-04-28), KAO CORP: "Detergent Composition" XP002413296 & JP 2005 113078 A (KAO CORP) 28. April 2005 (2005-04-28)
- DATABASE WPI Week 199831 Derwent Publications Ltd., London, GB; AN 1997-448329 XP002414732 GREFF M DANIEL: & ZA 9 709 135 A (STOA SA) 14. April 1998 (1998-04-14)

## Beschreibung

Die vorliegende Erfindung betrifft gelartige, polyacrylatverdickte und tensidhaltige Reinigungszubereitungen mit verbessertem Schaumverhalten.

Der Wunsch nach einem sauberen und gepflegten Äußeren ist wohl so alt wie die Menschheit. Unreine Haut und ein ungepflegtes Haarkostüm bieten idealen Nährboden und Heimstatt für Krankheitserreger und Parasiten aller Art. Die Lust an der Körperhygiene wurde stetig verstärkt, als in den 60er Jahren des 20. Jahrhunderts neben der "klassischen" Seife auch flüssige Reinigungsmittel mit neuentwickelten synthetischen Tensiden formuliert werden konnten. Baden und Duschen sind seitdem aus unserem täglichen Leben nicht mehr wegzudenken und den Verbrauchern stehen heutzutage eine Vielzahl von Produkten für die Reinigung der verschiedenen Körperpartien zur Verfügung.

Kosmetische und/oder dermatologische Reinigungspräparate werden in aller Regel in Form eines Schaums mit Wasser auf die zu reinigenden Körperpartien aufgetragen. Basis fast aller kosmetischen oder dermatologischen Reinigungspräparate sind waschaktive Tenside. Tenside sind amphiphile Stoffe, die organische, unpolare Substanzen in Wasser lösen können. Sie zeichnen sich durch ein ambivalentes Verhalten gegenüber Wasser und Lipiden aus: Das Tensidmolekül enthält mindestens je eine hydrophile und eine lipophile Gruppe, die die Anlagerung an der Grenzfläche zwischen diesen beiden Substanzklassen ermöglichen. Auf diese Weise sorgen Tenside für eine Herabsetzung der Oberflächenspannung des Wassers, die Benetzung der Haut, die Erleichterung der Schmutzentfernung und -lösung, ein leichtes Abspülen und - je nach Wunsch - auch für Schaumregulierung. Damit ist die Grundlage für die Schmutzentfernung lipidhaltiger Verschmutzungen gegeben.

Die hohe Reinigungsleistung tensidhaltiger Reinigungszubereitungen insbesondere gegenüber Lipiden, birgt jedoch auch eine Reihe von dermatologischen Nachteilen in sich: Bereits bei einer Reinigung der Haut mit Hilfe von Wasser - ohne Zusatz von Tensiden - kommt es zunächst zu einer Quellung der Hornschicht der Haut. Der Grad dieser Quellung hängt u. a. von der Dauer des Bades und dessen Temperatur ab. Gleichzeitig werden wasserlösliche Stoffe ab- bzw. ausgewaschen, wie z. B. wasserlösliche Schmutzbestandteile, aber auch hauteigene Stoffe, die für das Wasserbindungsvermögen der Hornschicht verantwortlich sind (sogenannte Feuchthaltemittel oder Moisturizer). Durch hauteigene oberflächenaktive Stoffe werden außerdem auch Hautfette in gewissem Ausmaß gelöst und ausgewaschen. Dies bedingt nach anfänglicher Quellung eine nachfolgende Austrocknung der Haut.

Es ist verständlich, dass waschaktive Tenside, die Haut und Haar von fettigen und wasserlöslichen Schmutzbestandteilen reinigen sollen, auch eine entfettende Wirkung auf die normalen Hautlipide haben. Bei jeder Hautreinigung werden in unterschiedlichem Maß auch interkorneozytäre Lipide und Sebumbestandteile entfernt. Das bedeutet, dass der natürliche Wasser-Lipid-Mantel der Haut bei jedem Waschvorgang mehr oder weniger gestört wird. Dies kann besonders bei extremer Entfettung zu einer kurzzeitigen Veränderung der Barrierefunktion der Haut führen, wobei selbstverständlich auch der jeweilige Zustand der behandelten Hautregion auf die dargestellten Veränderungen von erheblichem Einfluss ist. Beispielsweise kann die Hautdicke, die Anzahl der Talg- und Schweißdrüsen sowie die damit verbundene Empfindlichkeit erheblich variieren.

Grundsätzlich gilt dementsprechend als Forderung an waschaktive Tenside, dass sie biologisch möglichst inaktiv sind, um unerwünschte Nebenwirkungen zu vermeiden. Sie sollen ihre reinigende Wirkung bei optimaler Milde, bester Hautverträglichkeit und geringer Entfettung entfalten.

Es hat daneben aber auch nicht an Versuchen gefehlt, geeignete Reinigungszubereitungen zu finden, welche die Haut bei guter Reinigungsleistung gleichzeitig regenerieren bzw. "rückfetten". Allerdings bleibt die erzielte Leistung häufig hinter der erwarteten zurück, so dass der Anwender in aller Regel auf separate Pflegeprodukte zurückgreifen muss, welche nach der Reinigung auf die Haut aufgetragen werden und auf dieser verbleiben (sogenannte "leave-on" Produkte).

Eine besondere Gruppe an Hautreinigungsprodukten bilden dabei die Gesichtsreinigungsprodukte. Da die Gesichtshaut besonders empfindlich ist, werden für die Gesichtsreinigung besonders milde und die Haut nicht reizende Produkte eingesetzt. Meist werden dabei Gele, das heißt halbfeste, mehr oder weniger transparente Systeme verwendet.

Reinigungsgele enthalten als Hauptbestandteile Wasser, Tenside und Verdicker (Gelbildner).

Die Verdicker, auch Gelbildner genannt, bilden in dem Reinigungsgel ein dreidimensionales Netzwerk aus, in dem die Flüssigkeit (in der Regel Wasser) immobilisiert ist. Als Verdicker werden neben Salzen meist Polymere wie Polyacrylate eingesetzt. Diese werden der zu verdickenden Zubereitung bei neutralem pH-Wert zugesetzt und anschließend durch die Zugabe von Basen deprotoniert, wodurch die Zubereitung in ein viskoses Gel übergeht.

Grundsätzlich sollen Reinigungsgele sowohl mild und schonend zur Haut sein als auch einen feinporigen, cremigen Schaum in ausreichender Menge erzeugen. Ferner sollen sie möglichst transparent und klar sein, da sich so wirkungsvoll optische Effektstoffe einarbeiten lassen und transparente Zubereitungen vom Verbraucher aus ästhetischen Gründen bevorzugt werden.

Herkömmliche, nach dem Stand der Technik hergestellte Reinigungsgele weisen eine Reihe von Unzulänglichkeiten auf:
- Damit die Zubereitung mild wird und die Haut nicht zu stark entfettet, dürfen nur ausgewählte Tenside in geringer Konzentration in die Zubereitung eingearbeitet werden. Derartige Zubereitungen lassen sich aber nur unzureichend aufschäumen. Der Schaum ist nicht besonders feinporig und cremig und fällt schnell wieder in sich zusammen. Auch lässt sich die Viskosität von Zubereitungen mit einem geringen Tensidgehalt nicht auf herkömmliche Art und Weise durch den Zusatz von Salzen erhöhen.
- Um die Viskosität von Zubereitungen mit geringem Tensidgehalt zu erhöhen, müssen der Formulierung Polymere Verdicker (z.B. Carbopole) zugesetzt werden. Derartige Verdicker behindern aber die Schaumbildung der Zubereitung.
- Die Kombination aus herkömmlichen Tensiden und polymeren Verdickern führt in der Regel zur Eintrübung der Zubereitung. Optische Effekte, beispielsweise der Zusatz von Abrasiva oder Glitterstoffen, kommen nur unzureichend zur Geltung.
- Wichtig für eine einfache Anwendung des Produktes ist das Aufschäumverhalten. Darüber hinaus sollte das Produkt eine gelartiges Konsistenz aufweisen, d.h. es sollte nicht zu leicht fließen. Je höher die Viskosität ist, desto weniger dünnflüssig ist das Produkt. Idealerweise sollte das Produkt sich mit Wasser leicht vermischen, und dadurch leicht aufschäumen. Wenn ein Produkt zu viskös ist, ist das Aufschäumen mit Wasser schwieriger, da es sich nicht so gut mit Wasser vermischen lässt. Ein ideales Produkt wäre so dünnflüssig wie möglich, wiese aber noch eine gewisse Plastizität auf, hätte also eine Fliessgrenze.

Es war daher die Aufgabe der vorliegenden Erfindung, die Mängel des Standes der Technik zu beseitigen und milde Reinigungsgele mit großer Schaumkraft und hoher Transparenz zu entwickeln. Zusätzlich sollte eine besondere Langzeit-Lagerstabilität erreicht werden.

Überraschend wurde gefunden, dass der Zusatz von 1,2-Alkandiolen zu den erfindungsgemäßen Reinigungsprodukten insbesondere das Schaumvermögen und die Transparenz der Zubereitung erheblich verbessert.

Als besonders geeignet hat sich das 1,2-Hexandiol herausgestellt.

Der Einsatz von 1,2-Hexandiol in kosmetischen Zubereitungen ist dem Fachmann an sich bekannt, doch konnte der Stand der Technik nicht den Weg zur vorliegenden Erfindung weisen.

Die WO 95/01151 offenbart die Verwendung von Alkandiolen mit 5-7 Kohlenstoffatomen in kosmetischen Zubereitungen. Erfindungsgemäße Zubereitungen mit 1,2-Hexandiol sind jedoch nicht explizit offenbart.

Die DE 103 41 179 offenbart Deodorantzusammensetzungen mit einer Kombination aus Alkan-1,2-diolen, u. a. 1,2-Octandiol und α- und/oder β-Hydroxysäuren. Erfindungsgemäße Zubereitungen mit 1,2-Hexandiol sind jedoch nicht offenbart.

EP 1078638 offenbart Lichtschutzzubereitungen mit hohen Konzentrationen an 1,2-Hexandiol.

JP 2005113067 A und JP 2005113078 A offenbaren Reinigungszubereitungen, die Phosphorsäuremonoester und -diester als reinigende Tenside beschreiben. Bestimmte Alkandiole werden diesen Reinigungszubereitungen zugesetzt. Derartige Zubereitungen zeichnen sich u.a. durch sehr gute Schaumeigenschaften aus.

Zwar kennt der Stand der Technik Reinigungsgele enthaltend quervernetzte AcrylatCopolymere. So beschreiben die WO 01/076552, WO 01/019946, EP 1291000, EP 1291001, EP1291002, EP1291003 und EP 1291005 derartige Zubereitungen. Doch konnten diese Schriften nicht den Weg zur vorliegenden Erfindung weisen.

All diesen Schriften fehlt es an Hinweisen, das 1,2-Hexandiol hilfreich verwendet werden kann, um die Schaumeigenschaften von erfindungsgemäßen Zubereitungen zu verbessern.

Daher ergab es sich für den Fachmann überraschend und nicht vorhersehbar, dass eine kosmetische gelförmige waschaktive Zubereitung enthaltend ein oder mehrere anionischer Tenside, gewünschtenfalls weitere nichtionische, amphotere und/oder zwitterionische Tenside, ein oder mehrere gelbildende Acrylatverdicker gewählt aus der Gruppe der vernetzten Alkali-quellbaren Acrylatcopolymere, ein oder mehrere 1,2-Alkandiole mit 5 Kohlenstoffatomen, wobei die Zubereitung eine Fließgrenze von 0,5 - 20 Pa aufweist, bevorzugt 1 - 6 Pa, gemessen als kritische Schubspannung einer Fließkurve auf einem schubspannungsgesteuerten Rheometer bei 25°C +/- 1°C mit 25 mm Platte/Platte Geometrie bei einem Spalt zwischen 0,8 mm und 1,2 mm, wobei strukturschonend befüllt wird, eine geeignete konstante Schubspannungszeitrampe vorgegeben wird, vor dem Test eine entsprechende Strukturerholungszeit eingehalten wird und die kritische Schubspannung im Maximum der Fließkurve angegeben wird und wobei die Zubereitung eine Viskosität von 1500-10000 mPas aufweist und
eine kosmetische gelförmige waschaktive Zubereitung enthaltend ein oder mehrere anionischer Tenside, gewünschtenfalls weitere nichtionische, amphotere und/oder zwitterionische Tenside, ein oder mehrere gelbildende Acrylatverdicker gewählt aus der Gruppe der vernetzten Alkali-quellbaren Acrylatcopolymere, ein oder mehrere 1,2-Alkandiole mit 6 Kohlenstoffatomen, dadurch gekennzeichnet, dass das erfindungsgemäße Reinigungsgel einen oder mehrere Komplexbildner ausgewählt aus der Gruppe bestehend aus Weinsäure und deren Anionen, Citronensäure und deren Anionen, Aminopolycarbonsäuren und deren Anionen (wie beispielsweise Ethylendiamintetraessigsäure (EDTA) und deren Anionen, Nitrilotriessigsäure (NTA) und deren Anionen, Hydroxyethylendiaminotriessigsäure (HOEDTA) und deren Anionen, Diethylenaminopentaessigsäure (DPTA) und deren Anionen, trans-1,2-Diaminocyclohexantetraessigsäure (CDTA) und deren Anionen) in einer Gesamtkonzentration von 0,1 bis 2,0 Gew.-%, bezogen auf das Gesamtgewicht der Zubereitung, enthält und wobei die Zubereitung eine Fließgrenze von 0,5 - 20 Pa aufweist, bevorzugt 1 - 6 Pa, gemessen als kritische Schubspannung einer Fließkurve auf einem schubspannungsgesteuerten Rheometer bei 25°C +/- 1°C mit 25 mm Platte/Platte Geometrie bei einem Spalt zwischen 0,8 mm und 1,2 mm, wobei strukturschonend befüllt wird, eine geeignete konstante Schubspannungszeitrampe vorgegeben wird, vor dem Test eine entsprechende Strukturerholungszeit eingehalten wird und die kritische Schubspannung im Maximum der Fließkurve angegeben wird und wobei die Zubereitung eine Viskosität von 1500-10000 mPas aufweist, den Mängeln des Standes der Technik abhilft und insbesondere ein besonders ausgeprägtes Schaumvermögen der Zubereitung ermöglicht. Darüber hinaus werden eine gute Reinigungsleistung und gutes Hautgefühl erreicht. Zusätzlich wurde es überraschend gefunden, dass mit Zugabe von Alkandiolen es möglich ist, Reinigungsgele mit einer Fliessgrenze zu herstellen, die aber eine niedrige Viskosität als bisher haben.

Gemäß einer Ausführungsform ist das Alkandiol 1,2 Hexandiol. Besonders bevorzugt ist es, wenn mehr als 0,3 Gew.-%, ganz besonders bevorzugt mehr als 0,6 Gew.-%, ganz außergewöhnlich bevorzugt mehr als 0,8 Gew.-% Alkandiol enthalten sind. Weiter besonders bevorzugt ist es, wenn weniger als 3 Gew.-% Alkandiol enthalten sind. Besonders bevorzugt ist es auch, wenn die Zubereitung eine Transmission von 50% bis 100% bei einer Wellenlänge von 420 nm aufweist.

Solche Produkte weisen eine sehr gute Langzeitstabilität auf. Diese garantiert über den gesamten Verwendungszeitraum eine gleich bleibende Produktqualität und Produktleistung. Durch gute Schaum- und sehr gute Reinigungsleistung des Produktes ist die Anwendung eines zusätzlichen Körperreinigungsproduktes nicht mehr nötig. Sowohl objektiv nachvollziehbare als auch subjektiv spürbare Rückfettung generiert ein gepflegtes Hautgefühl. Dies ermöglicht Menschen mit normaler Haut auf zusätzliche rückfettende Körperpflegemittel, deren Einsatz nach Dusche und Bad sehr verbreitet ist, zu verzichten.

Die Zubereitung weist eine Viskosität von 1500 -10000 mPas auf. Solche emulsionsförmigen Zubereitungen weisen eine sehr attraktive optische Anmutung für den Anwender auf. Neben den Pflege- und/ oder Reinigungseigenschaften stellt die Handhabung eines kosmetischen Produktes einen für den Verbraucher sehr wichtigen Punkt dar. Die hier beschriebene Formulierung verfügt über sehr gute Anwendungseigenschaften. Sie lässt sich leicht entnehmen und dosieren. Zudem ist sie auf der Haut gut verteilbar und lässt sich nach der Anwendung einfach abspülen.

Die Erfindung umfasst auch eine Tube, einen Pumpspender oder eine Flasche enthaltend eine erfindungsgemäße Zubereitung. Besonders bevorzugt ist es, wenn das Verpackungsbehältnis transparent ist. Weiterhin umfasst die Erfindung ein Trägermaterial getränkt mit einer erfindungsgemäßen Zubereitung sowie die Verwendung von 1,2-Alkandiolen mit 5 oder 6 Kohlenstoffatomen zur Verbesserung des Schaumvermögens von tensidhaltigen gelförmigen waschaktiven Zubereitungen mit gelbildenden Acrylatverdickern.

Die erfindungsgemäße Zubereitung weist eine Fließgrenze von 0,5 - 20 Pa, bevorzugt 1 - 6 Pa, auf.

Als Fließgrenze wird die kritische Schubspannung der Fließkurve angesehen. Sie kann erfindungsgemäß wie folgt ermittelt werden:
Gemessen wird die Fließkurve auf einem schubspannungsgesteuerten Rheometer bei 25°C +/- 1°C mit 25 mm Platte/Platte Geometrie bei einem Spalt zwischen 0,8 mm und 1,2 mm, wobei strukturschonend befüllt wird. Es wird eine geeignete konstante Schubspannungszeitrampe vorgegeben und vor dem Test eine entsprechende Strukturerholungszeit eingehalten und die kritische Schubspannung im Maximum der Fließkurve angegeben.

Vorteilhaft werden die Zubereitungen so ausgestaltet, dass sie einen tan delta von 0,05 - 0,6 aufweisen, bevorzugt 0,1 - 0,5.

Unter tan delta wird erfindungsgemäß der Quotient aus dem Verlustmodul und dem Speichermodul verstanden. Der tan delta wird wie folgt ermittelt:
Gemessen werden Verlust- und Speichermodul durch einen dynamischen Frequenztest auf einem schubspannungsgesteuerten Rheometer bei 40 °C +/- 1°C mit 25 mm Platte/Platte Geometrie bei einem Spalt zwischen 0,8 mm und 1,2 mm, wobei strukturschonend befüllt wird. Es wird nach dem Stand der Technik der Frequenztest mit einer entsprechenden Strukturerholungszeit vor dem Test durchgeführt und der tan delta im Frequenzbereich zwischen 0,05 rad/s und 3,0 rad/s angegeben, bevorzugt zwischen 0,08 rad/s und 1,0 rad/s.

Die Fließgrenze kann durch Erhöhung der Gelbildnerkonzentration angehoben werden.

Die erfindungsgemäße Transmission wurde erfindungsgemäß mit dem Gerät Agilent 8453 Diode Array Spectrometer bei einer Schichtdicke von 1 cm bestimmt. Die Messung wurde in einem Bereich von 190 bis 1100 nm in Intervallen von 1 nm und einer Integrationszeit von 0,5 Sekunden bei Raumtemperatur durchgeführt. Erfindungsgemäß bevorzugt beträgt die Transmission der erfindungsgemäßen Zubereitung von 50% bis 100% bei 420 nm Wellenlänge.

Auch ist es erfindungsgemäß vorteilhaft, wenn das Reinigungsgel einen pH-Wert von pH 6 bis pH 8 aufweist.

Erfindungsgemäß bevorzugte Ausführungsformen der vorliegenden Erfindung sind dadurch gekennzeichnet, dass die Zubereitung einen oder mehrere Konservierungsstoffe in einer Konzentration von 0,1 bis 2,0 Gew.-% und besonders bevorzugt von 0,2 bis 1,2 Gew.-%, jeweils bezogen auf das Gesamtgewicht der Zubereitung, enthält.

Vorteilhafte Konservierungsmittel im Sinne der vorliegenden Erfindung sind beispielsweise Formaldehydabspalter (wie z. B. DMDM Hydantoin, welches beispielsweise unter der Handelsbezeichnung Glydant™ von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfasst das Konservierungssystem erfindungsgemäß ferner vorteilhaft auch Konservierungshelfer, wie beispielsweise Octoxyglycerin, Glycine Soja etc. Diese Liste der vorteilhaften Konservierungsmittel soll keineswegs limitierend sein. Vielmehr sind alle für Kosmetika oder Lebensmittel zugelassenen Konservierungsmittel vorteilhaft im Sinne der vorliegenden Erfindung.

Erfindungsgemäß besonders bevorzugt ist dabei der Einsatz von Parabenen und Phenoxyethanol als Konservierungsmittel.

Das erfindungsgemäße Reinigungsgel, enthaltend ein Alkandiol mit 6 Kohlenstoffatomen oder optional ein Alkandiol mit 5 Kohlenstoffatomen, enthält einen oder mehrere Komplexbildner in einer Gesamtkonzentration von 0,1 bis 2,0 Gew.-% und besonders bevorzugt in einer Konzentration von 0,2 bis 1,0 Gew.-%, jeweils bezogen auf das Gesamtgewicht der Zubereitung.

Der oder die Komplexbildner wird aus der Gruppe bestehend aus Weinsäure und deren Anionen, Citronensäure und deren Anionen, Aminopolycarbonsäuren und deren Anionen (wie beispielsweise Ethylendiamintetraessigsäure (EDTA) und deren Anionen, Nitrilotriessigsäure (NTA) und deren Anionen, Hydroxyethylendiaminotriessigsäure (HOEDTA) und deren Anionen, Diethylenaminopentaessigsäure (DPTA) und deren Anionen, trans-1,2-Diaminocyclohexantetraessigsäure (CDTA) und deren Anionen) gewählt.

Erfindungsgemäß besonders bevorzugt ist es Hydroxyethylendiaminotriessigsäure und insbesondere das Natriumsalz der Hydroxyethylendiaminotriessigsäure (Na₃HEDTA) als Komplexbildner einzusetzen.

Es ist erfindungsgemäß vorteilhaft, wenn das erfindungsgemäße Reinigungsgel einen oder mehrere Hautbefeuchtungsmittel in einer Konzentration von 0,5 bis 10 Gew.-% und bevorzugt in einer Konzentration von 2,0 bis 6,0 Gew.-%, jeweils bezogen auf das Gesamtgewicht der Zubereitung, enthält.

Erfindungsgemäß vorteilhafte Hautbefeuchtungsmittel sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist.

Erfindungsgemäß besonders bevorzugt ist dabei der Einsatz von Glycerin als Hautbefeuchtungsmittel.

Es ist erfindungsgemäß vorteilhaft, wenn das erfindungsgemäße Reinigungsgel Abrasiva (z. B. Peelingpartikel aus Polyethylen), Glitterstoffe, Effektstoffe, Farbschlieren, Gasblasen (insbesondere Luftblasen), Perlglanzpigmente, Glimmer, Öl-und/oder Emulsionströpchen enthält.

Auch ist es vorteilhaft im Sinne der vorliegenden Erfindung, wenn das erfindungsgemäße Reinigungsgel einen oder mehrere Farbstoffe enthält. Dabei können insbesondere alle bekannten, wasserlöslichen und für die Kosmetik zugelassenen Farbstoffe der Zubereitung zugesetzt werden.

Selbstverständlich ist es erfindungsgemäß von Vorteil, dem erfindungsgemäßen Reinigungsgel weitere kosmetische Wirk-, Hilfs- und Zusatzstoffe zuzusetzen. Die Nachfolgende Aufzählung gibt eine kleine Auswahl erfindungsgemäß vorteilhafter weiterer Zusätze, die aber keinesfalls die vorliegende Erfindung auf diese Verbindungen beschränken soll.

Die wässrige Phase der erfindungsgemäßen Reinigungsgele kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Butylenglykol, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte, Perlglanzagentien, Antischuppenwirkstoffe, Pflanzenextrakte, Vitamine, Wirkstoffe.

Besonders vorteilhafte Zubereitungen werden ferner erhalten, wenn als Zusatz- oder Wirkstoffe Antioxidantien eingesetzt werden.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Carnosin, natürliche und/oder synthetische Isoflavonoide, Kreatin, Kreatinin, Taurin und/oder β-Alanin sowie 8-Hexadecen-1,16-dicarbonsäure (Dioic acid, CAS-Nummer 20701-68-2; vorläufige INCI-Bezeichnung Octadecendioic acid), Licochalcon A, Ascorbinsäure und deren Derivate Vitamin E und dessen Derivate, Vitamin A und dessen Derivate,y-Oryzanol, Panthenol und/oder Niacinamid.

Ein weiterer erfindungsgemäß vorteilhafter Wirkstoff stellt beispielsweise Polidocanol dar.

Die Menge der Wirkstoffe (eine oder mehrere Verbindungen) in den Zubereitungen beträgt erfindungsgemäß vorzugsweise 0,001 bis 30 Gew.-%, besonders bevorzugt 0,05 bis 20 Gew.-%, inbesondere 0,1 bis 10 Gew.-%, bezogen auf das Gesamtgewicht der Zubereitung.

Erfindungsgemäß vorteilhaft wird die erfindungsgemäße Zubereitung in einer Tube, einem Pumpspender oder einer Flasche aufbewahrt und aus dieser heraus angewendet. Derartige Verpackungsbehältnisse sind erfindungsgemäß bevorzugt transparent.

Daher sind auch Tuben, Pumpspender oder Flaschen enthaltend eine erfindungsgemäße Zubereitung, erfindungsgemäß, wobei transparente Verpackungsbehältnisse erfindungsgemäß bevorzugt sind.

Vorteilhaft im Sinne der vorliegenden Erfindung ist es ferner, das erfindungsgemäße Reinigungsgel auf ein Trägermaterial (z. B. ein Tuch, Pad, Wattebausch) aufzutragen. Derartige Träger bestehen vorzugsweise aus Viskose-, Baumwolle- und/oder Polyesterfasern.

Erfindungsgemäß ist daher auch ein Trägermaterial (z. B. ein Tuch, Pad, Wattebausch) getränkt mit einem erfindungsgemäßen Reinigungsgel, wobei das Trägermaterial bevorzugt aus Viskose-, Baumwolle- und/oder Polyesterfasern aufgebaut ist.

Die nachfolgenden Beispiele sollen die vorliegende Erfindung verdeutlichen ohne sie einzuschränken. Die Angaben beziehen sich stets auf Gew.-%, sofern nicht andere Angaben gemacht werden.

### Beispiele

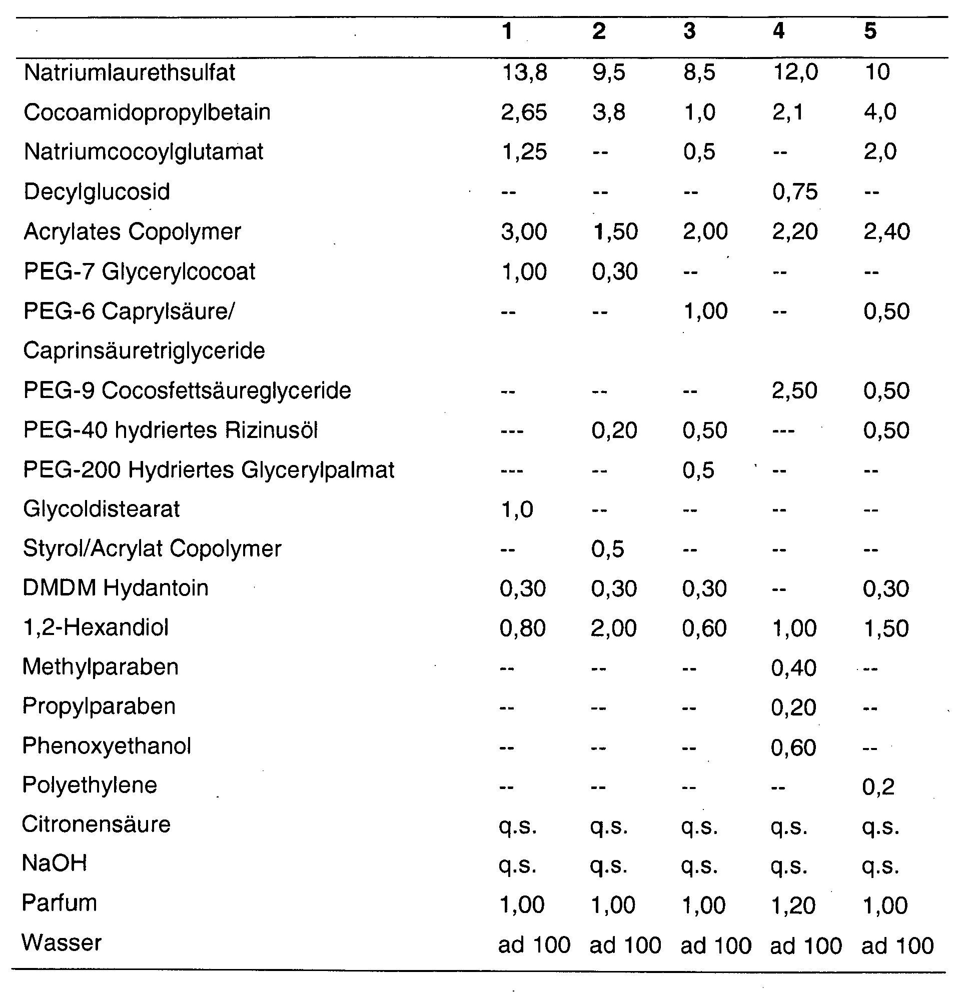

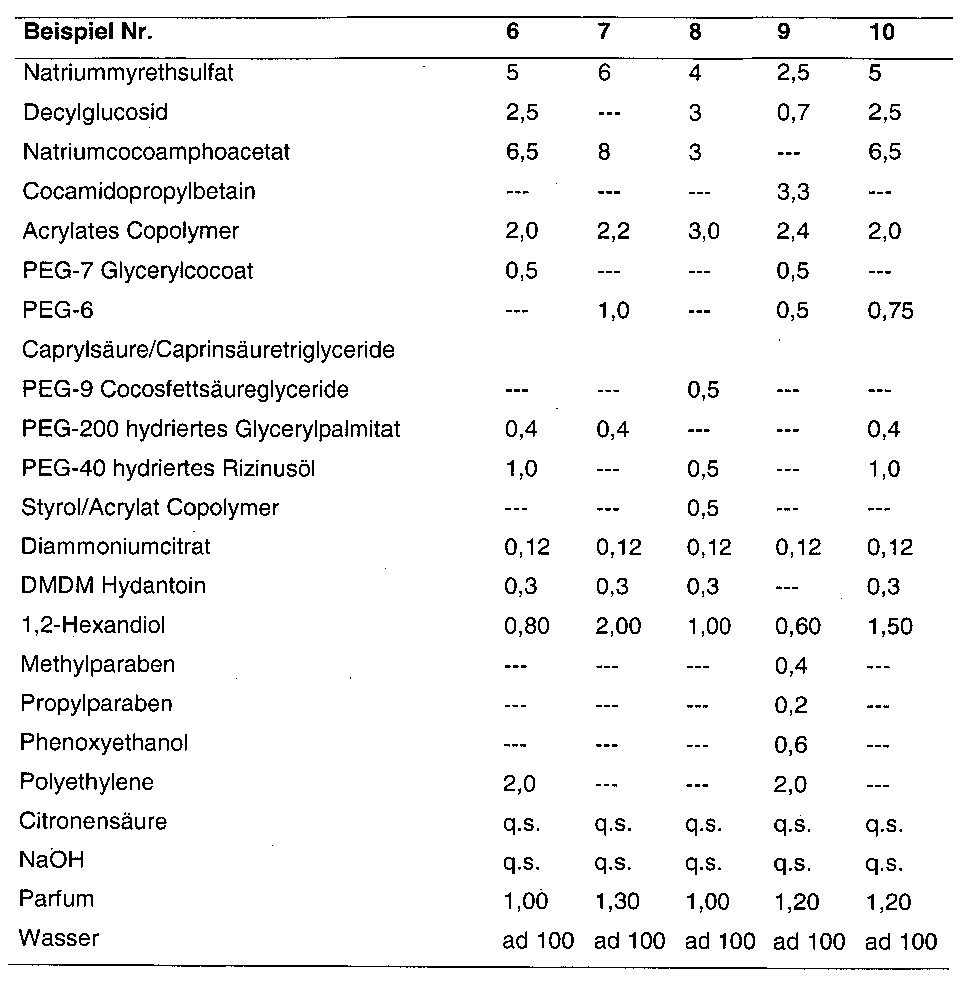

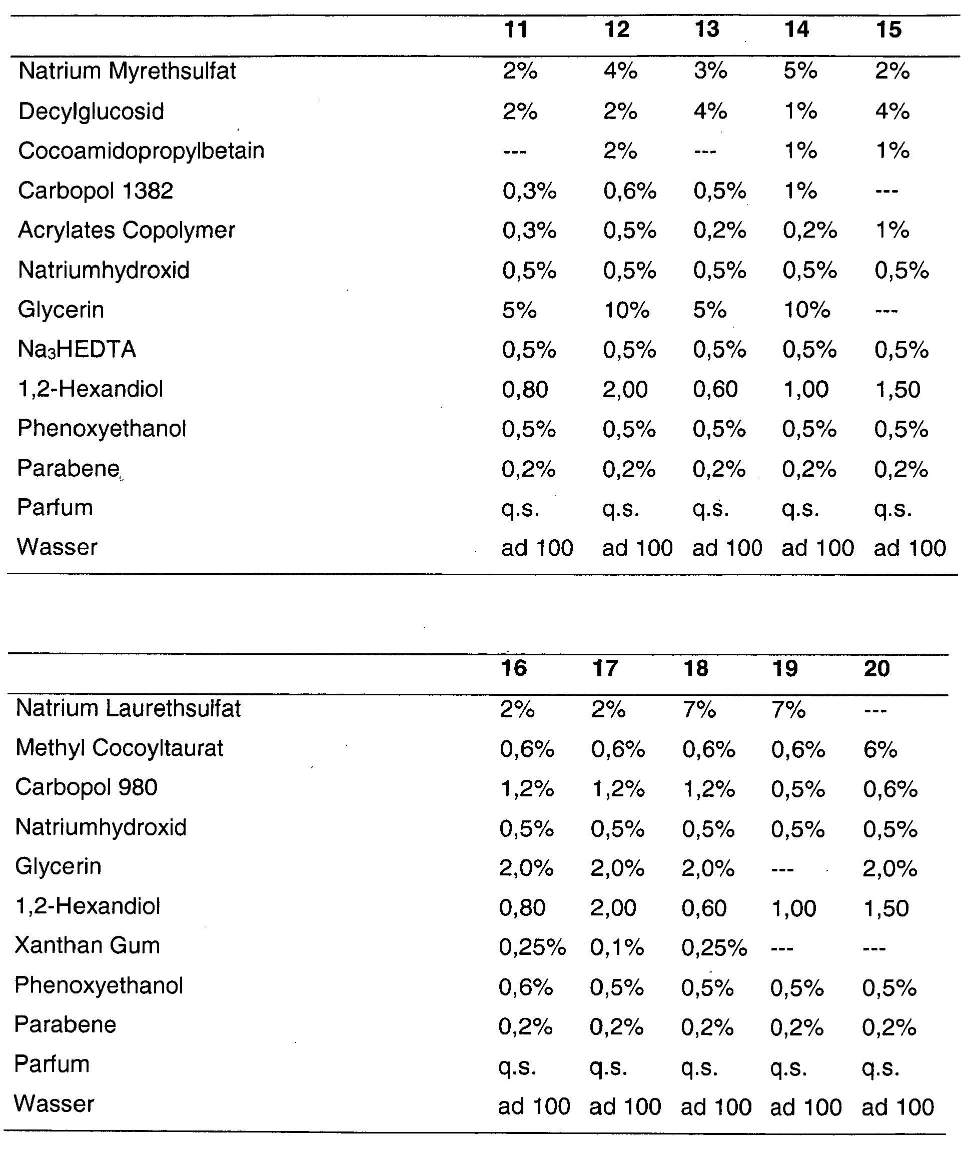

## Patentansprüche

1. Kosmetische gelförmige waschaktive Zubereitung enthaltend ein oder mehrere anionischer Tenside, gewünschtenfalls weitere nichtionische, amphotere und/oder zwitterionische Tenside, ein oder mehrere gelbildende Acrylatverdicker gewählt aus der Gruppe der vernetzten Alkali-quellbaren Acrylatcopolymere, ein oder mehrere 1,2-Alkandiole mit 5 Kohlenstoffatomen und
wobei die Zubereitung eine Fließgrenze von 0,5 - 20 Pa aufweist, bevorzugt 1 - 6 Pa, gemessen als kritische Schubspannung einer Fließkurve auf einem schubspannungsgesteuerten Rheometer bei 25°C +/- 1°C mit 25 mm Platte/Platte Geometrie bei einem Spalt zwischen 0,8 mm und 1,2 mm, wobei strukturschonend befüllt wird, eine geeignete konstante Schubspannungszeitrampe vorgegeben wird, vor dem Test eine entsprechende Strukturerholungszeit eingehalten wird und die kritische Schubspannung im Maximum der Fließkurve angegeben wird und
wobei die Zubereitung eine Viskosität von 1500-10000 mPas aufweist.

2. Kosmetische gelförmige waschaktive Zubereitung enthaltend ein oder mehrere anionischer Tenside, gewünschtenfalls weitere nichtionische, amphotere und/oder zwitterionische Tenside, ein oder mehrere gelbildende Acrylatverdicker gewählt aus der Gruppe der vernetzten Alkali-quellbaren Acrylatcopolymere, ein oder mehrere 1,2-Alkandiole mit 6 Kohlenstoffatomen, **dadurch gekennzeichnet, dass** das erfindungsgemäße Reinigungsgel einen oder mehrere Komplexbildner ausgewählt aus der Gruppe bestehend aus Weinsäure und deren Anionen, Citronensäure und deren Anionen, Aminopolycarbonsäuren und deren Anionen (wie beispielsweise Ethylendiamintetraessigsäure (EDTA) und deren Anionen, Nitrilotriessigsäure (NTA) und deren Anionen, Hydroxyethylendiaminotriessigsäure (HOEDTA) und deren Anionen, Diethylenaminopentaessigsäure (DPTA) und deren Anionen, trans-1,2-Diaminocyclohexantetraessigsäure (CDTA) und deren Anionen) in einer Gesamtkonzentration von 0,1 bis 2,0 Gew.-%, bezogen auf das Gesamtgewicht der Zubereitung, enthält und wobei
die Zubereitung eine Fließgrenze von 0,5 - 20 Pa aufweist, bevorzugt 1-6 Pa, gemessen als kritische Schubspannung einer Fließkurve auf einem schubspannungsgesteuerten Rheometer bei 25°C +/- 1°C mit 25 mm Platte/Platte Geometrie bei einem Spalt zwischen 0,8 mm und 1,2 mm, wobei strukturschonend befüllt wird, eine geeignete konstante Schubspannungszeitrampe vorgegeben wird, vor dem Test eine entsprechende Strukturerholungszeit eingehalten wird und die kritische Schubspannung im Maximum der Fließkurve angegeben wird und
wobei die Zubereitung eine Viskosität von 1500-10000 mPas aufweist.

3. Zubereitung nach Patentanspruch 2, **dadurch gekennzeichnet, dass** das Alkandiol 1,2-Hexandiol ist.

4. Zubereitung nach einem der vorhergehenden Ansprüche, **dadurch gekennzeichnet, dass** mehr als 0,3 Gew.-%, bevorzugt mehr als 0,6 Gew.-%, besonders bevorzugt mehr als 0,8 Gew.-% Alkandiol enthalten sind.

5. Zubereitung nach einem der vorhergehenden Ansprüche, **dadurch gekennzeichnet, dass** weniger als 3 Gew.-% Alkandiol enthalten sind.

6. Tube, Pumpspender oder Flasche enthaltend eine Zubereitung nach einem der vorhergehenden Ansprüche.

7. Verpackungsbehältnis nach Anspruch 6, **dadurch gekennzeichnet, dass** das Verpackungsbehältnis transparent ist.

8. Trägermaterial getränkt mit einer Zubereitung nach einem der vorhergehenden Ansprüche.

## Claims

1. Cosmetic gel-type wash-active preparation containing one or more anionic surfactants, optionally further non-ionic, amphoteric and/or zwitterionic surfactants, one or more gel-forming acrylate thickeners selected from the group of cross-linked alkali-swellable acrylate copolymers, one or more 1,2-alkanediols having 5 carbon atoms and
wherein the preparation has a yield point of 0.5-20 Pa, preferably 1-6 Pa, measured as the critical shear stress of a flow curve on a shear stress-controlled rheometer at 25°C +/- 1°C with 25 mm plate/plate geometry at a gap between 0.8 mm and 1.2 mm, wherein charging is carried out in a structure-preserving manner, a suitable constant shear stress gradient is predefined, before the test a corresponding structure recovery time is observed and the critical shear stress at the maximum of the flow curve is stated and wherein the preparation has a viscosity of 1500-10 000 mPas.

2. Cosmetic gel-type wash-active preparation containing one or more anionic surfactants, optionally further non-ionic, amphoteric and/or zwitterionic surfactants, one or more gel-forming acrylate thickeners selected from the group of cross-linked alkali-swellable acrylate copolymers, one or more 1,2-alkanediols having 6 carbon atoms, **characterized in that** the cleansing gel according to the invention comprises one or more complexing agents selected from the group consisting of tartaric acid and anions thereof, citric acid and anions thereof, aminopolycarboxylic acids and anions thereof (such as ethylenediaminetetraacetic acid (EDTA) and anions thereof, nitrilotriacetic acid (NTA) and anions thereof, hydroxyethylenediaminotriacetic acid (HOEDTA) and anions thereof, diethyleneaminopentaacetic acid (DPTA) and anions thereof, trans-1,2-diaminocyclohexanetetraacetic acid (CDTA) and anions thereof) at a total concentration of 0.1 to 2.0% by weight, based on the total weight of the preparation and
wherein
the preparation has a yield point of 0.5-20 Pa, preferably 1-6 Pa, measured as the critical shear stress of a flow curve on a shear stress-controlled rheometer at 25°C +/- 1°C with 25 mm plate/plate geometry at a gap between 0.8 mm and 1.2 mm, wherein charging is carried out in a structure-preserving manner, a suitable constant shear stress gradient is predefined, before the test a corresponding structure recovery time is observed and the critical shear stress at the maximum of the flow curve is stated and wherein the preparation has a viscosity of 1500-10 000 mPas.

3. Preparation according to Patent Claim 2, **characterized in that** the alkanediol is 1,2-hexanediol.

4. Preparation according to any of the preceding claims, **characterized in that** more than 0.3% by weight, preferably more than 0.6% by weight, particularly preferably more than 0.8% by weight alkanediol are present.

5. Preparation according to any of the preceding claims, **characterized in that** less than 3% by weight alkanediol are present.

6. Tube, pump dispenser or bottle containing a preparation according to any of the preceding claims.

7. Packaging container according to Claim 6, **characterized in that** the packaging container is transparent.

8. Support material impregnated with a preparation according to any of the preceding claims.

## Revendications

1. Préparation cosmétique détergente en forme de gel, contenant un ou plusieurs tensioactifs anioniques, éventuellement d'autres tensioactifs non ioniques, amphotères et/ou zwitterioniques, un ou plusieurs épaississants acrylate gélifiants choisis dans le groupe des copolymères d'acrylate réticulés gonflant dans un alcali, un ou plusieurs 1,2-alcanediols contenant 5 atomes de carbone,
la préparation présentant une limite d'écoulement de 0,5 à 20 Pa, de préférence de 1 à 6 Pa, mesurée en tant que contrainte de cisaillement critique d'une courbe d'écoulement sur un rhéomètre à contrainte de cisaillement contrôlée à 25 °C ± 1 °C avec une géométrie plaque/plaque de 25 mm avec une fente comprise entre 0,8 mm et 1,2 mm, le remplissage étant effectué de manière à préserver la structure, une rampe contrainte de cisaillement-temps constante appropriée étant fixée, un temps de repos de la structure approprié étant respecté avant le test, et la contrainte de cisaillement critique étant indiquée par le maximum de la courbe d'écoulement, et
la préparation présentant une viscosité de 1 500 à 10 000 mPas.

2. Préparation cosmétique détergente en forme de gel contenant un ou plusieurs tensioactifs anioniques, éventuellement d'autres tensioactifs non ioniques, amphotères et/ou zwitterioniques, un ou plusieurs épaississants acrylate gélifiants choisis dans le groupe des copolymères d'acrylate réticulés gonflant dans un alcali, un ou plusieurs 1,2-alcanediols contenant 6 atomes dé carbone, **caractérisée en ce que** le gel détergent selon l'invention contient un ou plusieurs complexants choisis dans le groupe constitué par l'acide tartrique et ses anions, l'acide citrique et ses anions, les acides aminopolycarboxyliques et leurs anions (tels que par exemple l'acide éthylène-diamine-tétraacétique (EDTA) et ses anions, l'acide nitrilotriacétique (NTA) et ses anions, l'acide hydroxyéthylène-diaminotriacétique (HOEDTA) et ses anions, l'acide diéthylène-aminopentaacétique (DPTA) et ses anions, l'acide trans-1,2-diaminocyclohexane-tétraacétique (CDTA) et ses anions) en une concentration totale de 0,1 à 2,0 % en poids, par rapport au poids total de la préparation,
la préparation présentant une limite d'écoulement de 0,5 à 20 Pa, de préférence de 1 à 6 Pa, mesurée en tant que contrainte de cisaillement critique d'une courbe d'écoulement sur un rhéomètre à contrainte de cisaillement contrôlée à 25 °C ± 1 °C avec une géométrie plaque/plaque de 25 mm avec une fente comprise entre 0,8 mm et 1,2 mm, le remplissage étant effectué de manière à préserver la structure, une rampe contrainte de cisaillement-temps constante appropriée étant fixée, un temps de repos de la structure approprié étant respecté avant le test, et la contrainte de cisaillement critique étant indiquée par le maximum de la courbe d'écoulement, et
la préparation présentant une viscosité de 1 500 à 10 000 mPas.

3. Préparation selon la revendication 2, **caractérisée en ce que** l'alcanediol est le 1,2-hexanediol.

4. Préparation selon l'une quelconque des revendications précédentes, **caractérisée en ce que** plus de 0,3 % en poids, de préférence plus de 0,6 % en poids, de manière particulièrement préférée plus de 0,8 % en poids d'alcanediol est contenu.

5. Préparation selon l'une quelconque des revendications précédentes, **caractérisée en ce que** moins de 3 % en poids d'alcanediol est contenu.

6. Tube, distributeur à pompe ou bouteille, contenant une préparation selon l'une quelconque des revendications précédentes.

7. Contenant d'emballage selon la revendication 6, **caractérisé en ce que** le contenant d'emballage est transparent.

8. Matériau support imprégné avec une préparation selon l'une quelconque des revendications précédentes.
